# EUROPEAN PATENT APPLICATION

(11) **EP 0 830 854 A2**
(43) Date of publication of application: **25.03.1998**
(21) Application number: 97112886.3
(22) Date of filing: 29.03.1994
(51) Int. Cl.: A61F 2/42

(54) **Apparatus for removal of osteal prothesis**

(62) Divisional of application: 94302231.9
(71) Applicant: Young, Michael John Radley, Ashburton, Devon TQ13 7JX (GB); BRADNOCK, Brian Robert Denis Peter, Hemel Hempstead, HP3 0PD (GB)
(72) Inventor: Young, Michael John Radley, Ashburton, Devon TQ13 7JX (GB); BRADNOCK, Brian Robert Denis Peter, Hemel Hempstead, HP3 0PD (GB)
(74) Representative: Gregory, Timothy Mark

(57) **Abstract**

The invention provides means for dislodging an installed osteal prostesis such as hip joint implanted in a femur.

A claw probe attachment (81) may be used to melt and extract the cement in small quantities around the pheriphery of the end of the fragment to provide the enlarged opening (53). The tool comprises a stem (82) having at its distal end a small saucer shaped cup (81), having apertures (83) therethrough, through which cement melted by untrasonic vibration of the cup (81), driven through the stem (82), may pass. The tool can be withdrawn periodically to extract cement or other material and thereby to form the enlarged opening. In this way, it functions as a scraper to provide or enlarge a space allowing engagement of an end of the broken prosthesis by the extraction tool. Providing space allows, a tool having a larger spoon shaped end may be inserted alongside the prosthesis to engage beneath its distal end.

Having thus prepared the enlargement (53) in bone cement at the proximal end of femur (52), and the depth d₁ beyond the breake responsible for the embedded fragment (50), all is in readiness for use of a special tool bit Fig. 7 as a replacement for the chuck or collet element (12) in any of the embodiments described.

## Description

The invention relates to an ultrasonic method and means for removing an osteal prosthesis from cemented installation in a living bone, as in the course of revision arthroplasty.

It is known from U.S. **Patents 4,248,232 and 5,151,099** that ultrasound may be used to facilitate removal of bone cement (PMMA) during revision arthroplasty. Local heating, by preferential absorption of ultrasound energy, raises the temperature of a small volume of the cement above the glass-transition temperature, thus allowing the cement to flow and to be manipulated into a shape and form which may be readily removed from the revision site.

It has also been claimed (**Hood, et al., U.S. Patent 5,045,054**) that the application of ultrasound directly to the prosthesis can break the bond between the prosthesis and surrounding cement or, in the case of uncemented prostheses, between the prosthesis and in-grown cancellous bone. The direction of applied ultrasonic energy is in line with the central axis of an implanted prosthetic devise, such as the axis of stem support for the ball of a hip-joint replacement. But the in-line application of force, as in the context of the **Hood, et al.** system, is to require the patient to oppose the force, with inevitable trauma for the patient.

It is an object of the invention to provide an improved method and means for dislodging an installed osteal prosthesis with a minimum of trauma for the patient who is faced with the need of revision arthroplasty.

A specific object is to meet the above object for the case of revision arthroplasty of a hip-joint prosthesis that has been implanted and cemented in a femur.

It is a general object to meet the above objects with a method and apparatus of reliably high performance without a daunting level of operating complexity, which method and apparatus are applicable to the full variety of currently available prostheses.

The body annulus will also accommodate a special tool which may be selected from one of a group of chucks, each of which is configured to coupling to the buried end of a broken fragment of a prosthetic shaft, with an ability to transfer sufficient ultrasonic energy through the tool and into the broken and buried prosthetic fragment, for clean and efficient dislodging and extra-ction of the buried fragment.

Embodiments of the present invention will now be more particularly described by way of example and with reference to the accompanying drawings, in which:
**Fig. 1** is a front elevation of a tool element for removing small quantities of cement;
**Fig. 2** is a side elevation of the tool element shown in **Fig. 1 ;** and
**Fig. 3** is a plan view of the tool shown in **Figs. 1 and 2.**

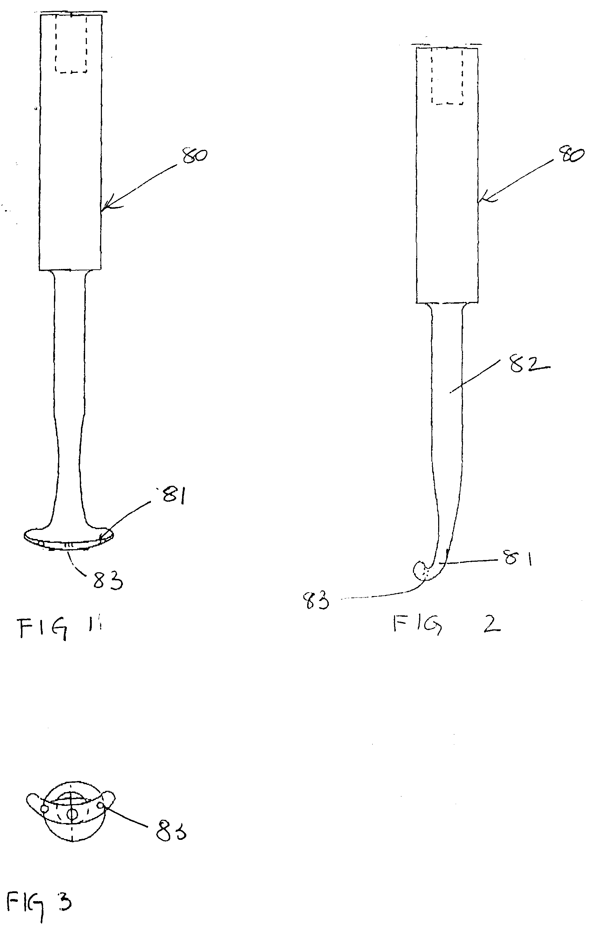

A tool 80 based on the technology described in the Patent Application number 94302231.9 is shown in **Figs 1 and 2**. A claw probe attachment 81 may be used to melt and extract the cement in small quantities around the periphery of the end of the fragment to provide the enlarged opening 53. The tool comprises a stem 82 having at its distal end a small saucer shaped cup 81, having apertures 83 therethrough, through which cement melted by ultrasonic vibration of the cup 81, driven through the stem 82, may pass. The tool can be withdrawn periodically to extract cement or other material and thereby to form the enlarged opening. In this way, it functions as a scraper to provide or enlarge a space allowing engagement of an end of the broken prosthesis by the extraction tool. Providing space allows, a tool having a larger spoon shaped end may be inserted alongside the prosthesis to engage beneath its distal end.

Having thus prepared the enlargement 53 in bone cement at the proximal end of femur 52, and the depth d₁ beyond the break responsible for the embedded fragment 50, all is in readiness for use of a special tool bit **Fig. 7** as a replacement for the chuck or collet element 12 in any of the embodiments described.

## Claims

1. A tool for use in revision arthroplasty wherein a prosthetic member to be removed is at least partially encumbered by bone cement, characterised in that it comprises a stem (82) connected to ultrasonic transducer means, said stem (82) having a spoon shaped distal end (81) provided with apertures through which cement melted by ultrasonic vibration of said tool may pass to be removed and in that said tool is so dimensioned that it is adapted to remove cement immediately adjacent said prosthetic member.
